# EUROPEAN PATENT SPECIFICATION

(11) **EP 0 286 221 B1**
(45) Date of publication and mention of the grant of the patent: **07.09.1994**
(21) Application number: 88301746.9
(22) Date of filing: 29.02.1988
(51) Int. Cl.: A61M 5/14, B05D 7/06

(54) **Drip infusion rate control apparatus**
Vorrichtung zur Steuerung der Infusionsgeschwindigkeit
Appareil de commande du débit de perfusion

(30) Priority: 27.02.1987 JP 46217/87; 03.08.1987 JP 193996/87; 28.08.1987 JP 215918/87; 28.08.1987 JP 215919/87
(43) Date of publication of application: 12.10.1988
(73) Proprietor: SHIMADZU CORPORATION, Kyoto 604 (JP); Nadai, Tanekazu, Fushimi-ku Kyoto (JP)
(72) Inventor: Nadai, Tanekazu, Fushimi-ku Kyoto (JP); Kawara, Toshio, Uji-Shi Kyoto (JP)
(74) Representative: Smith, Philip Antony

(56) References cited:
- EP-A- 0 100 682
- FR-A- 1 468 210
- GB-A- 2 054 200
- US-A- 4 405 318

## Description

The present invention relates to an apparatus to automatically control the infusion rate or speed of a drip infusion system.

As a method of injecting a medical solution into a living body, a drip infusion system is well known, in which a medical solution contained in a medicine bottle is gravitationally infused into an object through an infusion tube provided at one end with an infusion needle, the tube connecting between the bottle and the object with the needle thrusted into the body. Midway of the infusion tube there are provided in series a transparent drip chamber and a flow rate regulation clamp. The flow rate of the medical solution being infused is manually controlled with the clamp adjusted depending on intuition and experience in accordance with the size and frequency of droplets seen falling in the drip chamber. In spite of the fact that the speed of infusion is possibly an important factor in medical treatment, it is difficult or troublesome to obtain an optimum infusion condition by means of such a manual flow rate control made depending on intuition and experience. Further, it is inconvenient that the clamping is necessarily readjusted for a temperature variation, because the infusion speed depends on temperature through the temperature dependence of the viscosity and surface tension of the medical solution.

The present invention aims at improving the above mentioned inconveniences and disadvantages accompanying a simple drip infusion system, and makes it an object to provide a drip infusion rate apparatus, which automatically controls the flow rate of the medical solution being infused into a living body by a drip infusion system.

British Patent Specification GB-A-2054200 describes apparatus for this purpose in which the weight of a container holding the solution is sensed and a signal representing this actual weight is compared with a reference signal representing the ideal weight resulting from infusion at the rate necessary to dispense a desired amount over a specified period of time, any difference resulting from the comparison being used to adjust the flow-rate regulating clamp.

European Patent Specification EP-A-0100682 similarly monitors the weight of the container from which the solution is dispensed. The loss of weight over the time from commencement of infusion provides a measure of the actual delivery rate and this is compared with a pre-set delivery rate, the result of the comparison again being used to adjust the rate of delivery of the solution.

In accordance with the present invention there is provided a drip infusion rate control apparatus for controlling the flow rate of a medical solution into a human or animal body, the apparatus comprising a weighing mechanism for measuring the weight of a medicine container from which the solution flows to the body through an infusion tube, a flow control mechanism for increasing or decreasing the flow rate of the medicine from the container through the tube in response to an input control signal, a clocking means for measuring a period of time passing from the initiation of infusion into a human or animal body, a first memory for storing as paired data the periods of time measured by the clocking means and the weights measured by the weighing mechanism taken in as time goes, and an input means for inputting an aiming weight of a medicine to be infused and an aiming period of time required for infusion, a second memory for storing the input contents, and a data processing means for supplying a control signal to the flow control mechanism on the basis of the contents of the first memory and the second memory, characterized in that the data processing means comprises a first arithmetic means for calculating the current flow rate of the medicine on the basis of the data stored in the first memory, a second arithmetic means for calculating the subsequent remaining flow rates on the basis of the current remaining weight and the current remaining period of time corresponding to the aiming weight and the aiming period of time, a comparison and subtraction mechanism for comparing the values obtained by the first and second arithmetic means, the resulting difference being used as input to the flow control mechanism for adjusting the current flow rate of the medicine from the container to the remaining flow rate.

Embodiments of the invention also include a function for temporarily stopping the control operation for the purpose of complying, for instance, with a condition in which a proper control operation is made impossible because of the medicine bottle and infusion tube violently rolling and swinging owing to transferring the infusion system together with a patient receiving the infusion.

This function can be put into action by a manual switching operation.

Thus the infusion rate control can be temporarily stopped to avoid an erroneous control operation possibly arising, for instance, when the infusion system is transferred together with a patient receiving the infusion. Further, the infusion operation can also be stopped automatically with an alarm raised when the infusion rate happens to deviate from a predetermined allowable range for some reason or other.

The present invention is described in further detail in the following on reference to the accompanying drawing, in which:
Figure 1 is a block diagram showing the fundamental functional constitution of an apparatus similar to that described in EP-A-0100682;
Figure 2 shows a computerized electronic constitution for the apparatus of Figure 1;
Figure 3 is a block diagram showing the functional constitution of an embodiment of the present invention;
Figure 4 is a flow chart showing the operation of the embodiment of Figure 3;
Figure 5 shows a common appearance of the present invention applied to a drip infusion system;
Figure 6 shows a flow rate regulation mechanism to be used in the present invention; and
Figure 7 shows another flow rate regulation mechanism to be used in the present invention.

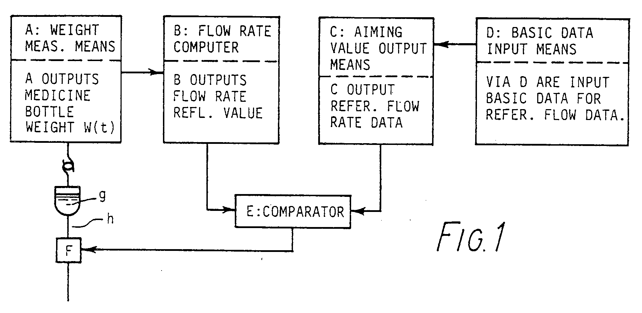

Referring to Figure 1, which illustrates the fundamental constitution of the apparatus, a weight measuring means A outputs a time-dependently varying weight W(t) of a medicine bottle g, from which a medical solution is being infused into a (not shown) patient through an infusion tube h. The varying weight W(t) is inputted to a flow rate computing means B, which computes from W(t) a value reflecting an existing medical solution flow rate. On the other hand, a set of reference flow rate data which defines an aiming flow rate is outputted from an aiming value outputting means C. Any data necessary for the aiming value outputting means C to output the set of reference flow rate data are manually inputted through a basic data inputting means D. The existing flow rate reflecting value outputted from the flow rate computing means is compared, at a comparator E, with the set of reference flow rate data outputted from the aiming value outputting means C. Then the comparator C outputs a flow rate regulation signal to a flow rate regulation mechanism F interposed midway of the infusion tube g. The flow rate regulation mechanism finally controls the flow rate of the medical solution at a predetermined aiming value.

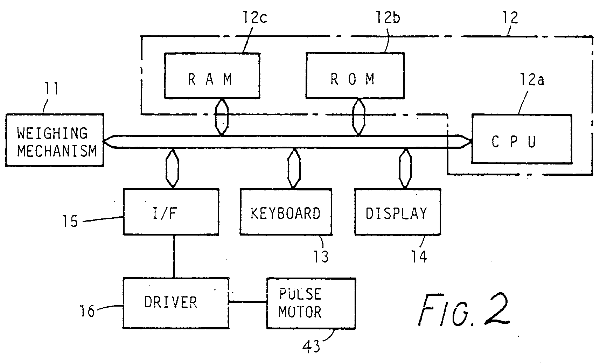

Such a functional constitution is practiced in a computerized electronic system as shown in Fig. 2. In the figure, a weighing scale 11 for weighing a medicine bottle g containing a medical solution consists, for example, of a load cell and an amplifier combined with an A-D converter. A series of time-dependently varying weight data W(ti) (ti: time) outputted from the weighing scale 11 is transferred to a micro-computerized arithmetic control part 12, which includes a CPU 12a to practice an arithmetic program and to control instruments concerned, a ROM 12b, and a RAM 12c. The RAM 12c has a first, a second and a third area. The first area always stores n successive weight data W(ti) from the weighing scale 11 with the oldest datum shifted each time a newest weight datum is inputted. Each time the newest datum is taken in, the n weight data are subjected to an arithmetic operation of averaging. The thus obtained successive average values are transferred, as proper values Wj (j: integers) of the time-dependently varying weight of the medicine bottle g (Figure 2(A)), to the second area, which always accepts m values of Wj (W1, W2, ....Wm) with the oldest value W1 shifted each time a newest value Wm+1 is taken in as W1. Further, each time the second area is thus renewed, a difference $\text{dW = W1-W2}$ is calculated as a value representing the average infusion flow rate in the time period To defined by (m-1) times the (constant) time interval between two adjacent calculation operations of Wj and Wj+1. Incidentally the time period To is usually selected to be several minutes. On the other hand the third area stores as an aiming infusion flow rate a weight value Wa to be infused in the same time period as To. The weight value Wa is inputted through a keyboard 13 manually in advance. The above weight difference dW representing an existing flow rate is compared with the weight value Wa representing an aiming flow rate. The result of comparison is outputted through an interface 15 to a driver 16 of a pulse motor 43, which is attached to an infusion flow rate regulation mechanism F (refer to Figure 1) devised so as to adjust the clamping degree of the infusion tube h. The infusion flow rate regulation mechanism F is later described in detail in conjunction with the description of an outward appearing setup of the embodiment. In the comparison of dW with Wa, if dW exceeds Wa, the driver 16 drives the pulse motor 43 to squeeze the infusion tube; if dW is smaller than Wa, the pulse motor 43 is driven to loosen the clamping of the infusion tube; and if dW is equal to Wa, the pulse motor 43 is not driven, and the degree of clamping the tube is kept unchanged. The above described operation is carried out in accordance with a program stored in the ROM 12b.

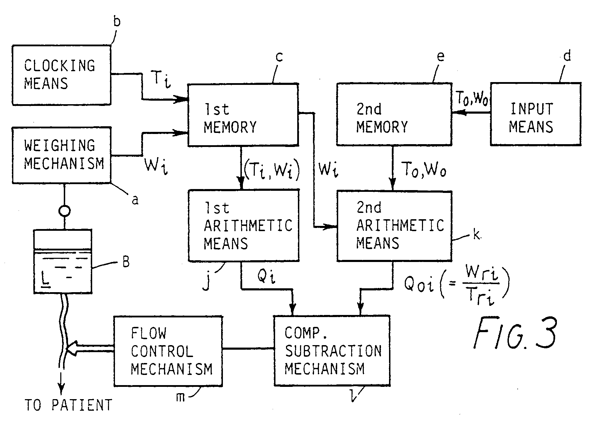

An embodiment of the present invention is described in the following with reference to Figure 3, which shows the functional constitution of the embodiment. In this embodiment, though the parts for deriving an existing flow rate and the reference flow rate data are similar to those of the apparatus of Figure 1, the aiming value outputting means (corresponding to C in Figure 1) consists of a memory e and an aiming flow rate computing means k. The memory e stores, as an aiming flow rate defining data, a total weight Wo to be infused and an aiming time period To. The aiming flow rate computing means k first derives a remaining weight from the weight data Wᵢ outputted from the weighing scale and then computes, from the above derived remaining weight, a flow rate Qₒᵢ necessary for the remaining weight to be completely infused at the end of the aiming time period To.

Thus in this embodiment the data processing means comprises a first arithmetic means j for calculating the current flow rate Qᵢ of the medicine on the basis of the data stored in the first memory c, a second arithmetic means k for calculating the subsequent remaining flow rates Qₒᵢ on the basis of the current remaining weight Wᵣᵢ and the current remaining period of time Tᵣᵢ corresponding to the aiming weight Wₒ and the aiming period of time Tₒ, a comparison and subtraction mechanism 1 for comparing the values obtained by the first and second arithmetic means, the resulting difference being used as input to the flow control mechanism m for adjusting the current flow rate Qi of the medicine from the container B to the remaining flow rate.

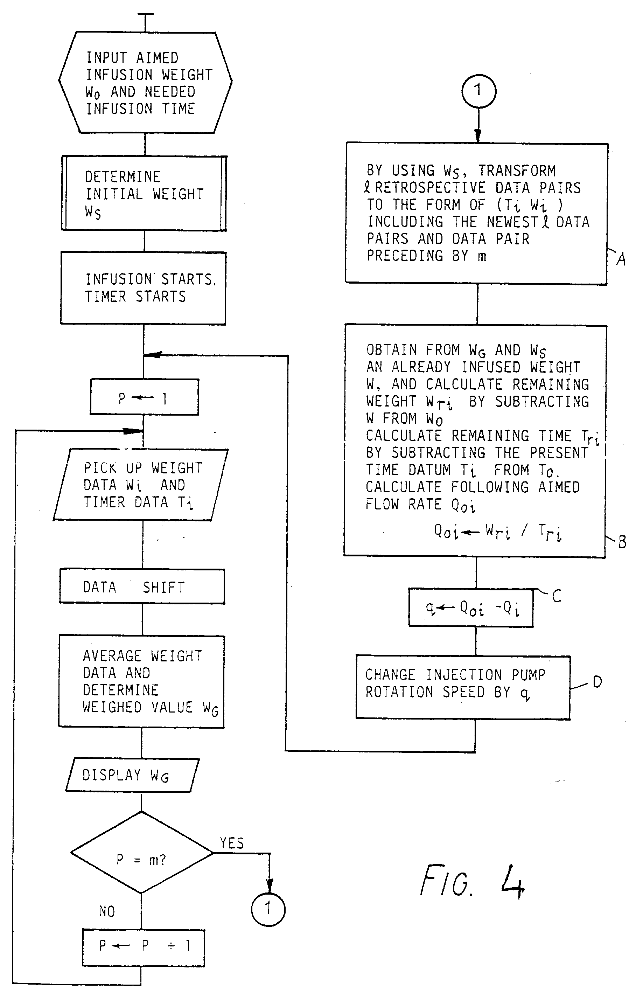

The functional constitution described is practised in a computerized electronic system similar to that of Figure 2 but using the program shown in Figure 4.

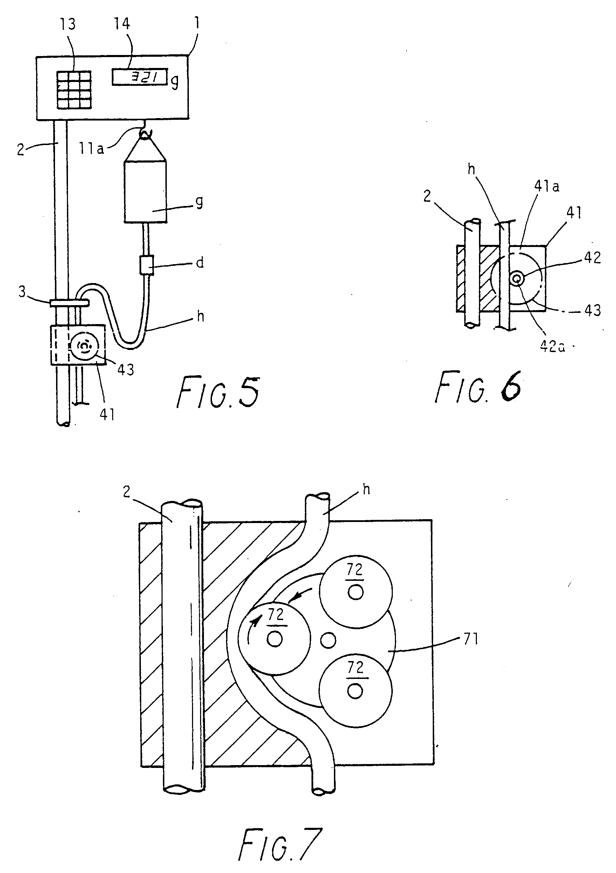

The outward appearance of the embodiment is shown in Figure 5, which illustrates a common appearance of the present invention applied to a usual drip infusion system. A housing 1, which is mounted on top of the pole 2 of a drip infusion system, encloses therein the electronic part shown in Figure 3 including the weighing scale 11 with the keyboard 13 and the display 14 made to appear on the front panel of the housing 1. The medicine bottle g of the drip infusion system is hanged down on a hook 11a having a mechanical connection to the weighing scale 11 hidden by the housing 1. From the medicine bottle g is led the infusion tube h to a (not shown) patient through the flow rate regulation mechanism F. Midway of the infusion tube h there may be provided a drip chamber d. The detail of the flow rate regulation mechanism F is described on reference to Fig. 6, which shows a cross-sectional view of the mechanism. Referring to Figure 6, the flow rate regulation mechanism F consists of a holder 41 having a cut-in groove 41a, an eccentric cam 42 provided in the groove 41 and the pulse motor 43 for rotating the cam 42. Between the bottom of the groove 41a and the eccentric cam 42 is inserted the infusion tube h. In such a constitution of the flow rate regulation mechanism F, the degree of throttling the infusion tube h is varied depending on the rotation angle of the eccentric cam 42. The electric wiring to the pulse motor 43 from the driver 16 hidden by the housing 1 is not shown in Figure 5.

Further, the above embodiment can be modified by adding an alarming means for alarming in an emergency and/or means for stopping the control operation.

## Claims

1. A drip infusion rate control apparatus for controlling the flow rate of a medical solution into a human or animal body, the apparatus comprising a weighing mechanism (a) for measuring the weight of a medicine container (B) from which the solution flows to the body through an infusion tube, a flow control mechanism (m) for increasing or decreasing the flow rate of the medicine from the container (B) through the tube in response to an input control signal, a clocking means (b) for measuring a period of time passing from the initiation of infusion into a human or animal body, a first memory (c) for storing as paired data the periods of time (Tᵢ) measured by the clocking means (b) and the weights (Wᵢ) measured by the weighing mechanism (a) taken in as time goes, and an input means (d) for inputting an aiming weight (Wₒ) of a medicine to be infused and an aiming period of time (Tₒ) required for infusion, a second memory (e) for storing the input contents, and a data processing means for supplying a control signal to the flow control mechanism (m) on the basis of the contents of the first memory and the second memory,
characterized in that the data processing means comprises a first arithmetic means (j) for calculating the current flow rate (Qᵢ) of the medicine on the basis of the data stored in the first memory (c), a second arithmetic means (k) for calculating the subsequent remaining flow rates (Qₒᵢ) on the basis of the current remaining weight (Wᵣᵢ) and the current remaining period of time (Tᵣᵢ) corresponding to the aiming weight (Wₒ) and the aiming period of time (Tₒ), a comparison and subtraction mechanism (1) for comparing the values obtained by the first and second arithmetic means, the resulting difference being used as input to the flow control mechanism (m) for adjusting the current flow rate (Qi) of the medicine from the container (B), to the remaining flow rate (Qₒᵢ).

## Patentansprüche

1. Vorrichtung zur Steuerung der Infusionsgeschwindigkeit zum Regeln der Strömungsgeschwindigkeit einer medizinischen Lösung in einen menschlichen oder tierischen Körper, wobei die Vorrichtung einen Wiegemechanismus (a) umfaßt, um das Gewicht eines Medizinbehälters (B) zu messen, von dem aus die Lösung durch einen Infusionsschlauch zu dem Körper fließt, einen Strömungsregelmechanismus (m) zum Verringern oder Erhöhen der Strömungsgeschwindigkeit der Medizin von dem Behälter (B) durch den Schlauch als Reaktion auf ein Eingangssteuersignal, eine Zeitgebereinrichtung (b) zum Messen der ab dem Beginn der Infusion in einen menschlichen oder tierischen Körper vergehenden Zeit, einen ersten Speicher (c) zum Speichern als Datenpaar der durch die Zeitgebereinrichtung (b) gemessenen Zeitspannen (Tᵢ) und der durch den Wiegemechanismus (a) im Verlauf der Zeit gemessenen Gewichte (Wᵢ), und eine Eingabeeinrichtung (d) zum Eingeben eines Zielgewichts (Wₒ) der einzuleitenden Medizin und einer Zielzeitspanne (Tₒ), die für die Infusion benötigt wird, einen zweiten Speicher (e) zum Speichern des Eingabeinhalts, und eine Datenverarbeitungseinrichtung zum Liefern eines Steuersignals an den Strömungsregelmechanismus (m) auf der Basis des Inhalts des ersten Speichers und des zweiten Speichers,
dadurch gekennzeichnet, daß die Datenverarbeitungseinrichtung eine erste arithmetische Einrichtung (j) umfaßt, die zum Berechnen der augenblicklich vorhandenen Strömungsgeschwindigkeit (Qᵢ) der Medizin auf der Basis der in dem ersten Speicher (c) gespeicherten Daten dient, eine zweite arithmetische Einrichtung (k) zum Berechnen der nachfolgend verbleibenden Strömungsgeschwindigkeiten (Qₒᵢ) auf der Basis des augenblicklich verbleibenden Gewichts (Wᵣᵢ) und der augenblicklich verbleibenden Zeitspanne (Tᵣᵢ) entsprechend dem Zielgewicht (Wₒ) und der Zielzeitspanne (Tₒ), einen Vergleichs- und Subtraktionsmechanismus (1) zum Vergleichen der durch die erste und zweite arithmetische Einrichtung gewonnenen Werte, wobei die resultierende Differenz als Eingang für den Strömungsregelmechanismus (m) dient, um die augenblickliche Strömungsgeschwindigkeit (Qᵢ) der Medizin von dem Behälter (B) aus auf die verbleibende Strömungsgeschwindigkeit (Qₒᵢ) einzustellen.

## Revendications

1. Appareil de commande du débit de perfusion goutte-à-goutte pour contrôler le débit d'une solution médicale dans un corps humain ou animal, l'appareil comportant un mécanisme de pesage (a) pour mesurer le poids du réceptable (B) de médicament à partir duquel la solution s'écoule dans le corps à travers un tube de perfusion, un mécanisme de commande de l'écoulement (m) pour augmenter ou diminuer le débit du médicament provenant du réceptable (B) à travers le tube, en réponse à un signal de commande d'entrée, un moyen d'horloge (b) pour mesurer une période de temps qui s'écoule depuis le début de la perfusion dans un corps humain ou animal, une première mémoire (c) pour stocker comme données appariées les périodes de temps (Tᵢ) mesurées par le moyen d'horloge (b) et les poids (Wᵢ) mesurés par le mécanisme de pesage (a), données recueillies à mesure que le temps s'écoule, et un moyen d'entrée (d) pour entrer un poids prescrit (Wₒ) d'un médicament devant être perfusé et une période de temps prescrite (Tₒ) nécessaires pour la perfusion, une deuxième mémoire (e) pour stocker les contenus de données entrées, et un moyen de traitement des données pour envoyer un signal de commande au mécanisme de commande d'écoulement (m) sur la base du contenu de la première mémoire et de la deuxième mémoire,
caractérisé en ce que le moyen de traitement des données comporte un premier moyen arithmétique (j) pour calculer le débit courant (Qᵢ) du médicament sur la base des données stockées dans la première mémoire (c), un deuxième moyen arithmétique (k) pour calculer le débit résultant (Qₒᵢ) sur la base du reliquat de poids courants (Wᵣᵢ) et du reliquat de la période de temps courante (Tᵣᵢ) correspondant au poids prescrit (Wₒ) et à la période de temps prescite (Tₒ), un mécanisme de comparaison et de soustraction (1) pour comparer les valeurs obtenues par les premier et deuxième moyens arithmétiques, la différence résultant étant utilisée comme entrée dans le mécanisme de commande d'écoulement (m) pour régler le débit courant (Qᵢ) de médicament provenant du réceptable (B) en fonction du résultant (Qₒᵢ).
